# FASCICULE DE BREVET EUROPEEN

(11) **EP 0 887 072 B1**
(45) Date de publication et mention de la délivrance du brevet: **09.01.2002**
(21) Numéro de dépôt: 98401360.7
(22) Date de dépôt: 08.06.1998
(51) Int. Cl.: A61K 7/48

(54) **Utilisation de sulfites et métabisulfites, pour la fabrication de compositions cosmétiques ou pharmaceutiques, notamment en dermatologie, à effet inhibiteur de la mélanogénèse ou à activité dépigmentante.**
Verwendung von Sulfiten und Metabisulfiten zur Herstellung von kosmetischen oder pharmazeutischen, insbesondere dermatologischen Zusammensetzungen, welche einen inhibitorischen Effekt auf die Melanogenese oder eine hautdepigmentierende Wirkung besitzen
Use of sulfites and metabisulfites for the manufacturing of cosmetic or pharmaceutical, in particular dermatological compositions having an inhibitory effect on melanogenesis or a skin whitening activity

(30) Priorité: 09.06.1997 FR 9707103
(43) Date de publication de la demande: 30.12.1998
(73) Titulaire: COLETICA, 69007 Lyon (FR)
(72) Inventeur: Bresson-Rival, Delphine, 69007 Lyon (FR); Perrier, Eric, 38138 Les Cotes d'Arey (FR)
(74) Mandataire: Portal, Gérard

(56) Documents cités:
- EP-A- 0 296 923
- EP-A- 0 419 901
- FR-A- 2 088 848
- FR-A- 2 736 263
- US-A- 4 847 075
- US-A- 5 773 014
- STN, Serveur de bases de données, XP002053246
- STN, Serveur de bases de données, XP002053247

## Description

La présente invention concerne essentiellement l'utilisation de sulfites ou métabisulfites, en cosmétique ou pour la fabrication d'une composition pharmaceutique, notamment dermatologique, à effet inhibiteur de la mélanogénèse ou à activité dépigmentante.

L'invention couvre également des compositions cosmétiques ou des compositions pharmaceutiques, notamment dermatologiques, à effet inhibiteur de la mélanogénèse ou à activité dépigmentante ainsi obtenue.

L'invention couvre encore un procédé de traitement cosmétique de dépigmentation utilisant les sulfites ou métabisulfites, en tant qu'actifs vis-à-vis de la dépigmentation.

On sait que pour lutter contre les rayonnements solaires, la peau possède des cellules différenciées particulièrement adaptées à cette fonction : les mélanocytes.

Au cours d'un procédé complexe, la mélanogénèse, ceux-ci fabriquent un pigment sombre, la mélanine, qui a pour effet de protéger les structures cutanées et d'augmenter le temps nécessaire pour contracter un érythème solaire.

Cependant, toutes les mélanines ne sont pas protectrices et il existe en particulier une forme de mélanine, appelée phaeomélanine qui est extrêmement phototoxique. Capable comme toutes les mélanines de réagir avec certaines formes de radicaux libres, elle provoque la formation de radicaux libres encore plus toxiques, susceptibles de causer des dommages irréversibles sur le matériel génétique des kératinocytes.

D'autre part, certains désordres liés à un dysfonctionnement de l'unité de mélanisation, sont susceptibles de provoquer une hyper pigmentation parfois particulièrement inesthétique.

Ainsi, l'utilisation d'inhibiteurs de la synthèse de mélanine est particulièrement intéressante en cosmétologie, non seulement pour des applications où une véritable dépigmentation est recherchée comme dans le cas du blanchiment de peau fortement pigmentée ou l'inhibition de l'hyper pigmentation dans certains aspects inesthétiques par exemple, mais également pour des applications visant à éclaircir le teint, à donner de la luminosité à la peau, de l'éclat aux tissus de surface.

Cette inhibition de la synthèse de mélanine peut être aussi particulièrement intéressante dans le cadre de traitement thérapeutique pour traiter une véritable pathologie.

Cependant, l'invention vise de préférence une simple utilisation cosmétique bien qu'une utilisation pharmaceutique dans le cadre de traitement de pathologie puisse également être envisagé et fasse aussi partie de l'invention.

### Art antérieur

Il a déjà été proposé diverses compositions cosmétiques ou éventuellement pharmaceutiques.

Comme art antérieur on pourra citer à titre d'exemple, le document GB-A-1349955 de 1974, décrivant une composition blanchissante de la peau à action synergique contenant de l'hydroquinone, un agent desquamant-irritant spécialement un ester d'acide gras à chaîne longue d'un acide gras insaturé, des alkylamines, et un agent corticostéroide anti-inflammatoire telle que la fluorométholone, la deaméthasone ou l'hydrocortisone.

D'autre part, le document roumain RO-81370, abrégé WPIL, AN-83-762909 est relatif à une crème émolliante pour enlever les taches de couleur de la peau à base de cétazole, de vaseline, de lanoline, d'un excipient comprenant des agents stabilisants anti-oxydants comme le métabisulfite (Na₂S₂O₅).

Egalement, le document JP-A-58198421, abrégé WPIL, AN-84-002869, décrit une composition blanchissante de la peau pour le traitement des maladies de la peau, contenant une huile de foie, une composition basique contenant des composé soufrés, une composition comprenant de l'eau oxygénée, de l'acide salicylique et une composition acide contenant un bromate. L'huile de foie peut être obtenue à partir de l'huile de foie de poisson, les exemples de composés soufrés sont l'acide thioglycolique, l'acide thiolactique, l'acide thiopropionique, l'acide thiosalicylique et l'acide thiobutyrique, de préférence sous forme de sel avec diverses amines ou le bisulfite de sodium et la cystéine. Le thioglycolate d'ammonium est spécialement préféré.

Par ailleurs, il est encore connu par le document US-A-4,692,261 une préparation dermique pour application externe ayant une activité d'inhibition de la mélanogénèse comprenant de l'acide kojique ou un dérivé d'acide kojique ainsi que du métabisulfite ou hydrogénosulfite de sodium et/ou du peroxyde d'hydrogène.

Il est précisé que la décoloration de la préparation est évitée pendant ou après le stockage pendant une longue période de temps par une incorporation d'hydrogénosulfite de sodium et/ou de peroxyde d'hydrogène de façon à maintenir la valeur commerciale de la préparation.

De ce fait, l'utilisation d'hydrogénosulfite de sodium ou métabisulfite est seulement envisagée en tant qu'agent stabilisant de la coloration de la préparation et non pas en tant qu'agent actif pour inhiber l'activité de la tyrosinase, enzyme fondamentale de la mélanogénèse.

Encore, le document-JP-A-04/082834 de 1992, abrégé WPIL AN-92-138630, décrit un médicament contenant de la galactosamine ou un sel et/ou de la mannosamine ou un sel avec divers excipients contenant des agents stabilisants dont l'hydrogénosulfite de sodium ou métabisulfite à une proportion de 0,05%.

Encore, le document JP-A-07/025742 de 1995 est relatif à une composition cosmétique blanchissante de la peau contenant des dérivés d'acide ascorbique, des dérivés d'hydroquinone et/ou des extrait de plantes et/ou des extraits de placenta comme principe actif ainsi que des agents stabilisants contenant du soufre et notamment un sel d'hydrogénosulfite afin d'empêcher la coloration de la préparation.

En résumé, l'art antérieur a utilisé le métabisulfite ou hydrogénosulfite seulement comme agent antidécoloration ou agent stabilisant mais en aucun cas comme principe actif inhibiteur de la melanogénèse.

Le document WO-A-9819665 est un document intercalaire publié le 14 mai 1998, postérieurement à la date de priorité du 9 juin 1997, qui décrit l'utilisation de sulfite comme agent dépigmentant.

Le document US-A-4,847,075 décrit un agent absorbant comprenant une substance réductrice supportée sur un silicate d'aluminium hydraté. Cette substance réductrice est une substance décomposant un pigment de mélanine pour absorber des complexes métalliques précieux tels que des complexes d'or, d'argent ou similaires (colonne 1, lignes 5 à 11 et les revendications).

Le document STN, serveur de base de données, XP-002053246 décrit des préparations cosmétiques dépigmentantes contenant de la glabridine et un sulfite comme agent antioxydant.

Le document FR-A-2736263 décrit une composition dermocosmétique dépigmentante contenant l'association d'un mélange acide comprenant au moins un acide α-hydroxylé et/ou un de ses dérivés, au moins un autre composé acide choisi parmi l'acide kojique, caféique, azélaïque, aminobutyrique, fusarique, 5-hydroxy 2-hydroxyméthyl-γ-pyridone, et leurs dérivés, au moins un extrait végétal d'au moins une plante parmi lesquelles notamment mûrier blanc, citron.

Une composition préférée comprend de 1 à15% d'acide kojique, de 20 à 30% d'acide glycolique et de 20 à 35% d'extraits végétaux, et le complément en excipient acceptable pour cette composition (page 4, lignes 18 à 24).

Les exemples 1 et 3 prévoient un stabilisant antioxydant tel que sulfite de sodium ou métabisulfite de sodium en une quantité inférieure à 1%.

Le document EP-A-0 419 901 décrit une préparation dermique pour application externe à base d'acide kojique ou d'un dérivé comme agent inhibiteur de mélanogénèse avec de l'hydrogénosulfite de sodium et/ou du peroxyde d'hydrogène comme agent inhibiteur de décoloration, c'est-à-dire comme agent antioxydant. La proportion en hydrogénosulfite de sodium est très inférieure à 1% dans les exemples.

Le document EP-A-0 296 923 Moet-Hennessy décrit une composition de liposomes contenant un extrait de mûrier en une proportion comprise entre 0,005 et 1% relativement au poids total de la composition (page 3, lignes 57 à 60).

La présence de sulfite de sodium comme agent antioxydant est envisagée dans l'exemple 4 relatif à des liposomes sous forme de poudre qui sont ensuite dilués dans l'utilisation sous forme de composition pharmaceutique ou cosmétique. L'exemple 4 ne donne pas lieu à une formulation pharmaceutique ou cosmétique. Les autres exemples donnent l'emploi de la poudre de l'exemple 1 respectivement dans des proportions de 1 à 4 grammes pour 100 grammes ce qui divise environ par 100 les quantités de produit utilisé.

Le document STN serveur de base de donnée XP-002053247 décrit une composition topique contenant du métabisulfite comme agent antioxydant d'acide tranexamique comme agent dépigmentant,

Le document FR-A-2.088.848 décrit un produit détacheur par décoloration des éléments tachants utilisant du sulfite de sodium comme agent antioxydant dans une composition ne contenant pas d'extrait de plante.

### Résumé de l'invention

L'invention a pour but principal de résoudre le problème consistant en la fourniture d'une composition cosmétique ou avantageusement aussi d'une composition pharmaceutique, notamment dermatologique, à effet inhibiteur de la mélanogénèse, permettant d'obtenir une activité dépigmentante pour un usage cosmétique et avantageusement indépendamment pour un usage pharmaceutique et/ou dermatologique dans le cadre de pathologie de la pigmentation.

La présente invention a encore pour but de résoudre le nouveau problème technique consistant en la fourniture d'une composition cosmétique et avantageusement une composition pharmaceutique, notamment dermatologique, à effet inhibiteur de la tyrosinase, enzyme fondamentale de la mélanogéaèse qui soit particulièrement stable notamment dans les domaines de pH préférés par l'homme de l'art qui travaille dans le domaine cosmétique, c'est-à-dire des pH compris entre environ 5 et environ 8.

L'invention a encore pour but de résoudre les nouveaux problèmes techniques énoncés ci-dessus, à l'aide d'une solution qui permet d'obtenir des compositions cosmétiques et avantageusement pharmaceutiques, notamment dermatologiques, à très grande stabilité, et sans émission d'odeur.

La présente invention a encore pour but de résoudre de nombreux problèmes techniques énoncés ci-dessus, selon une solution particulièrement simple, nécessitant un faible nombre de principes actifs inhibiteurs de mélanogénèse, en quantité limitée, qui soit particulièrement aisée à mettre en oeuvre et pouvant être fabriquée à l'échelle industrielle et cosmétique ou avantageusement aussi pharmaceutique, notamment dermatologique.

L'ensemble de ces problèmes techniques est résolu pour la première fois simultanément par la présente invention.

Ainsi, dans le cadre de l'invention, il a été découvert de manière particulièrement inattendue que les sulfites, incluant les métabisulfites, présentent une activité d'inhibition de la tyrosinase, enzyme fondamentale de la melanogénèse en procurant aux formulations qui les contiennent une activité dépigmentante particulièrement intéressante, principalement pour une utilisation cosmétique mais aussi avantageusement permettant une utilisation pharmaceutique et notamment dermatologique . dans le cadre du traitement de pathologie de la peau.

Dans le cadre de l'invention, il a pu être démontré de manière inattendue que les sulfites, incluant les métabisulfites, permettent de prévenir une pigmentation excessive en intervenant sur une réduction de l'activité de la tyrosinase.

Ainsi, selon un premier aspect, la présente invention concerne l'utilisation de sulfites, incluant les métabisulfites, comme agent cosmétique à activité inhibitrice de la tyrosinase, enzyme fondamentale de la mélanogénèse, ou à activité dépigmentante, en combinaison avec au moins un extrait végétal en une quantité suffisante pour diminuer ou empêcher l'apparition d'odeur.

Selon un deuxième aspect, la présente invention couvre également l'utilisation de sulfites, incluant les métabisulfites, pour la fabrication de compositions pharmaceutiques et notamment dermatologiques à activité d'inhibition de la mélanogénèse, ou activité dépigmentante, en combinaison avec au moins un extrait végétal en une quantité suffisante pour diminuer ou empêcher l'apparition d'odeur.

Selon un troisième aspect, la présente invention couvre aussi une composition cosmétique ou une composition pharmaceutique, notamment dermatologique, telle que définie dans les revendications de composition.

Selon un quatrième aspect, la présente invention couvre aussi un procédé cosmétique d'inhibition de la mélanogénèse ou de dépigmentation, caractérisé en ce qu'il comprend l'application sur les zones concernées de la peau d'un animal, avantageusement d'un être humain, de manière topique, d'une composition contenant comme principe actif inhibiteur de la mélanogénèse ou de dépigmentation, une quantité efficace dans ce but d'au moins un sulfite, incluant un métabisulfite, en combinaison avec au moins un extrait végétal, en une quantité suffisante pour diminuer ou empêcher l'apparition d'odeur.

Dans le cadre de la présente invention, que ce soit dans la description et les revendications, l'expression "sulfite" inclut tout sulfite et en particulier les sels de sulfite, en particulier, avec des métaux alcalins ou alcalinoterreux, de préférence avec des métaux alcalins tels que le sodium ou le potassium. Le sodium est actuellement davantage préféré.

D'autre part, l'expression inclut également des sels partiels de sulfite comme les métabisulfites ou hydrogénosulfites, en particulier, avec des métaux alcalins ou alcalinoterreux, de préférence avec des métaux alcalins et en particulier le sodium ou le potassium, de préférence le sodium.

Dans le cadre de l'un quelconque des aspects de l'invention, la proportion d'incorporation du sulfite est comprise entre 0,001% et 20%, avantageusement entre 0,01% et 10% en poids.

Par ailleurs, selon l'invention, les sulfites, incluant les métabisulfites, de la présente invention, sont utilisés en combinaison avec au moins un extrait végétal.

Cependant, il est préféré d'utiliser un extrait végétal choisi parmi le groupe consistant d'un extrait de mûrier, d'un extrait de citron, d'un extrait de saxifrage, d'un extrait de pamplemousse, d'un extrait de raisin ou d'un extrait d'oughon.

Les proportions d'incorporation de tels extraits varient dans de larges limites. La proportion totale d'extrait peut constituer la partie principale de la composition.

Généralement, la proportion en extrait sera comprise entre 0,01% et 90%, mieux entre environ 5% et 70% du poids total de la composition. On préfère utiliser des combinaisons d'extrait. Chaque extrait étant présent en des proportions d'au moins environ 5% et pas plus d'environ 30% afin d'utiliser au mieux les avantages procurés par chaque extrait, ces avantages étant bien apparents à un homme de l'art.

Il a pu être mis en évidence de manière particulièrement inattendu que la présence d'extraits végétaux résulte en une stabilisation efficace des sulfites incluant les métabisulfites.

Il a en effet été observé que les sulfites, incluant les métabisulfites, utilisés tels quels en solution cosmétique induisent l'apparition d'odeurs inacceptables d'un point de vue cosmétique, odeurs particulièrement sensibles à des pH compris entre 5 et 7 ou même à des pH plus acides.

Or, lorsque ces sulfites sont combinés à des extraits végétaux, ceux-ci masquent l'odeur dégagée par les sulfites, y compris lorsque ceux-ci sont placés à des pH classiques de la fomiulation cosmétique ou pharmaceutique, notamment dermatologique.

Par ailleurs, il a également pu être observé que les principes actifs ainsi réalisés sont parfaitement bien tolérés dans les applications cosmétiques et même avantageusement pharmaceutiques, notamment dermatologique, et présentent une cytotoxicité extrêmement faible, plus de 200 fois inférieur à celle des activités dépigmentantes habituellement utilisées et telle que exemplifiée par l'art antérieur précédemment énoncé, notamment celle utilisée en cosmétique comme l'hydroquinone ou l'acide kojique.

Il est à noter que les molécules de l'art antérieur ont leur activité qui repose sur l'observation d'un blanchiment de la peau très rapide basé sur la dégradation des structures organisées de la mélanine.

La plupart de ces produits se sont révélés corrosifs, cytotoxiques, voire sensibilisants, ce qui limite maintenant fortement leur utilisation en cosmétique.

Par ailleurs, si l'acide kojique, l'acide ascorbique et leurs dérivés sont extrêmement efficaces dans le blocage sélectif de l'activité de la tyrosinase, ces molécules sont très instables dans les milieux complexes que sont les formulations cosmétiques et s'oxydent rapidement en produits de décomposition colorés peu compatibles avec les caractères organoleptiques habituellement recherchés pour des formulations cosmétiques.

Par ailleurs, bien que sans aucune commune mesure avec celle observée pour l'hydroquinone et l'acide linoléique, une certaine toxicité est obtenue avec des produits de type acide kojique ou acide ascorbique.

Il a été découvert de manière particulièrement inattendue par les inventeurs que les sulfites, incluant les métabisulfites, non seulement sont très efficaces pour inhiber la tyrosinase, donc la mélanogénèse, et présentent ainsi une activité dépigmentante très importante, mais présentent aussi une absence de cytotoxicité ce qui constitue un avantage technique déterminant.

D'autre part, les sulfites, incluant les métabisulfites, présentent une excellente stabilité en fonction du pH et de la température en permettant de satisfaire des critères organoléptiques habituellement recherchés principalement par la cosmétique et aussi avantageusement en pharmacie et notamment en dermatologie, lorsqu'il sont combinés avec au moins un extrait végétal.

Naturellement, l'invention peut être combinée avec divers autres principes actifs à activité inhibitrice de la mélanogénèse ou non, ainsi qu'avec différents supports cosmétiquement ou pharmaceutiquement, notamment dermatologiquement, acceptables de façon à obtenir des formulations de crèmes ou de lotions susceptibles à éclaircir la peau sans aucun danger pour l'utilisateur. Ainsi, les compositions cosmétiques ou pharmaceutiques, notamment dermatologiques, peuvent être préparées pour avoir un pH compris entre environ 5 et environ 8.

Comme il a été indiqué précédemment, de telles compositions sont parfaitement stables et ne développent pas d'odeur même après plusieurs mois de stockage.

D'autres buts, caractéristiques et avantages de l'invention apparaissent clairement à l'aide de la description explicative qui fait référence à divers exemples d'essais d'efficacité et/ou de formulation sont donnés seulement à titre d'illustration. Dans les exemples, tous les modes de réalisation n'incluant pas d'extraits végétaux ne font pas partie de l'invention telle que revendiquée.

Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention dans sa fonction et dans sa généralité.

Ainsi, chaque exemple a une portée générale.

D'autre part, dans les exemples, tous les pourcentages sont donnés en poids, sauf indication contraire, la température ambiante est exprimée en degrées celsius, sauf indication contraire et la pression est la pression atmosphérique, sauf indication contraire.

### Exemple 1 de l'invention

### Essai in vitro de l'inhibition par le sulfite de sodium de la tyrosinase isolée

On sait que la tyrosinase est capable de catalyser la formation de L-Dopaquinone pui de dopachrome à partir de la L-Dopa. Or, le dopachrome est un composé coloré dont il est possible de suivre l'apparition par spectrophotométrie U.V. visible à 475 nm. L'utilisation d'un actif capable de modifier l'activité enzymatique va se traduire par une variation de la vitesse d'apparition de cette molécule colorée.
Le rapport des vitesses de formation du dopachrome permet de déterminer de façon précise les activations ou les inhibitions obtenues avec les différentes molécules testées.

Dans le cadre du présent essai in vitro, on a utilisé le sulfite de sodium dans ce test aux concentrations respectivement de 0,01%, 0,1%, 1% et 10% et les résultats obtenus sur l'inhibition de la tyrosinase sont exposés au tableau I ci-après:

**Tableau I**

| | | | | |
|---|---|---|---|---|
| Taux de concentration | 0,01% | 0,1% | 1% | 10% |
| Résultats (inhibition) | 93,7% | 100% | 100% | 100% |

Des tests organoleptiques ont encore été réalisés sur des solutions de sulfite de sodium à 5% sans ou avec soit 10% d'un extrait de mûrier soit 20% d'un extrait de citron. Ces tests ont été conduits pendant 4 semaines à 45°C au pH 5,5 et 6,5. Les résultats sont consignés dans le tableau II ci-dessous.

**Tableau II**

| 4 semaines à 45°C | | | |
|---|---|---|---|
| | Sulfite de Na (5%) | Sulfite de Na (5%) + extrait de mûrier (10%) | Sulfite de Na (5%) + extrait de citron (20%) |
| pH 5,5 | odeur extrêmement forte | absence d'odeur | absence d'odeur |
| pH 6,5 | odeur très forte | absence totale d'odeur | absence totale d'odeur |

Il résulte clairement du tableau I que le sulfite de sodium inhibe la quasi totalité de la tyrosinase même à une proportion aussi faible que 0,01% tandis que dans le tableau II on évite la présence d'une odeur en ajoutant un extrait de plante; par exemple un extrait de mûrier ou un extrait de citron, chacune de ces activités étant particulièrement inattendue pour un homme de l'art.

### Exemple 2 de l'invention

### Essai d'inhibition de la tyrosinase isolée par le métabisulfite de sodium

Dans ce test, on a utilisé le métabisulfite de sodium aux concentrations de 0,01%, 0,1%, 1% et 10% et les résultats obtenus sur l'inhibition de la tyrosinase sont exposés au tableau III ci-après :

**Tableau III**

| | | | | |
|---|---|---|---|---|
| Taux de concentration | 0,01% | 0,1% | 1% | 10% |
| Résultats (inhibition) | 92,8% | 100% | 100% | 100% |

On constate aussi que le métabisulfate de sodium a un effet quasi total d'inhibition de la tyrosinase même à une concentration aussi faible que 0,01%.

Egalement, des tests organoleptiques sont alors réalisés sur des solutions de métabisulfite de sodium à 5% sans ou avec soit 20% d'un extrait de saxifrage soit 10% d'un extrait de pamplemousse. Ces tests ont été conduits pendant 4 semaines à 45°C au pH 5,5 et 6,5. Les résultats sont consignés au tableau IV ci-après.

**Tableau IV**

| 4 semaines à 45°C | | | |
|---|---|---|---|
| | Métabisulfite de Na (5%) | Métabisulfite de Na (5%) + extrait de saxifrage (20%) | Métabisulfite de Na (5%) + extrait de pamplemousse (10%) |
| pH 5,5 | odeur extrêmement forte | absence d'odeur | absence d'odeur |
| pH 6,5 | odeur très forte | absence totale d'odeur | absence totale d'odeur |

Comme pour l'exemple 1, on constate que la présence d'un extrait de plante, ici un extrait de saxifrage ou un extrait de pamplemousse permet d'éviter la formation d'odeurs lors d'un stockage prolongé pendant 4 semaines à 45°C à un pH de 5,5 ou 6,5, ce qui est particulièrement inattendu pour un homme de l'art.

### Exemple 3 de l'invention

### Inhibition de la tyrosinase par le sulfite de potassium

Dans cet essai; il a été utilisé le sulfite de potassium aux concentrations de 0,01%, 0,1%, 1% et 10% et les résultats obtenus sur l'inhibition de la tyrosinase sont rapportés au tableau V ci-après :

**Tableau V**

| | | | | |
|---|---|---|---|---|
| Taux de concentration | 0,01 % | 0,1% | 1% | 10% |
| Résultats (inhibition) | 82,7% | 98,10% | 92,7% | 100% |

On constate à partir du tableau V ci-dessus que le sulfite de potassium permet d'obtenir une inhibition de plus de 80% même à une proportion aussi faible que 0,01%, une inhibition quasi complète dès proportions de 0,01%, ce qui est remarquable.

Des tests organoleptiques ont aussi été réalisés sur des solutions de sulfite de potassium à 5%, sans ou avec soit 20% d'un extrait de raisin, soit 10% d'un extrait d'oughon. Il est à noter que l'oughon est aussi dénommé Scutelaria. Ces tess ont été conduits pendant 4 semaines à 45°C au pH 5,5 et 6,5. Les résultats sont rapportés au tableau VI ci-après :

**Tableau VI**

| 4 semaines à 45°C | | | |
|---|---|---|---|
| | Sulfite de K (5%) | Sulfite de K (5%) à extrait de raisin (20%) | sulfite de K (5%) à extrait d'oughon (10%) |
| pH 5,5 | odeur extrêmement forte | absence d'odeur | absence d'odeur |
| pH 6,5 | odeur très forte | absence d'odeur | absence d'odeur |

Ici encore, on constate que la présence d'un extrait de plante, ici un extrait de raisin ou un extrait d'oughon, permet d'éliminer la présence d'une odeur lors d'un stockage prolongé à un pH de 5,5 ou de 6,5.

### Exemple 4 de l'invention

### Essai d'inhibition de la tyrosinase par un métabisulfite de potassium

Dans cet essai, le métabisulfite de potassium a été utilisé aux concentrations de 0,01%, 0,1%, 1% et 10% et les résultats obtenus sur l'inhibition de la tyrosinase sont rapportés au tableau VII ci-après :

**Tableau VII**

| | | | | |
|---|---|---|---|---|
| Taux de concentration | 0,01% | 0,1% | 1% | 10% |
| Résultats (inhibition) | 91,81% | 93,63% | 100% | 100% |

On constate à partir du tableau VII que le métabisulfite de potassium inhibe la tyrosinase à plus de 90%, soit quasi totalement, dès une concentration aussi faible que 0,01% en poids, ce qui est encore remarquable.

Des tests organoleptiques sont alors réalisés sur des solutions de métabisulfite de potassium à 5%, sans ou avec soit 10% d'un extrait de saxifrage, soit 5% d'un extrait d'oughon + 10% d'un extrait de pamplemousse. Ces tests ont été conduits également pendant 4 semaines à 45°C au pH 5,5 et 6,5. Les résultats sont rapportés au tableau VIII ci-après :

**Tableau VIII**

| 4 semaines à 45°C | | | |
|---|---|---|---|
| | Métabisulfite de K (5%) | Métabisulfite de K (5%) + extrait de saxifrage (10%) | Métabisulfite de K (5%) + extrait d'oughon (5%) + extrait de pamplemousse (10%) |
| pH 5,5 | odeur extrêmement forte | absence d'odeur | absence d'odeur |
| pH 6,5 | odeur très forte | absence totale d'odeur | absence totale d'odeur |

Il en ressort clairement du tableau VIII que la présence d'un extrait de plante, par exemple un extrait de saxifrage ou un extrait d'oughon combiné à un extrait de pamplemousse, permet d'éviter la présence d'une odeur lors d'un longue conservation à un pH 5,5 et 6,5 à une température de 45°C.

### Exemple 5 de l'invention

### Exemple de formulation d'une composition utilisable principalement à titre de composition cosmétique mais pouvant aussi être utilisée comme composition pharmaceutique ou dermatologique

On prépare la composition suivante, de manière classique, à partir des ingrédients actifs suivants :
- sulfite de sodium 5%
- métabisulfite de sodium 5%
- extrait de saxifrage 25%
- extrait de raisin 30%
- extrait de mûrier 5%
- extrait d'oughon 5%
- EDTA 0,5%
- eau déminéralisée QSP 100%

Il a été mesuré l'activité anti-tyrosinase, pour différentes concentrations de cette composition, par simple dilution avec de l'eau déminéralisée.
a) à une concentration de 0,015%, la composition inhibe déjà 21,6% d'activité tyrosinase;
b) à une concentration de 0,02%, la composition inhibe 46% de l'activité tyrosinase;
c) à 0,5% la composition inhibe 93% de l'activité tyrosinase.
d) à 1%, la composition inhibe 97% de l'activité tyrosinase
e) à une concentration égale ou supérieure à 5%, la composition inhibe totalement l'activité tyrosinase.

Cette composition utilisée pure présente une excellente stabilité à température ambiante et à 45°C. Après 4 semaines de stabilité à 45°C, les compositions ne présentent aucune odeur aux deux pH considérés, c'est-à-dire à pH 5,5 et pH 6,5.

Cette composition peut donc constituer une composition de base ou finale d'une composition cosmétique ou avantageusement aussi d'une composition pharmaceutique et notamment dermatologique à activité anti tyrosinase ou d'inhibition de mélanogénèse ou encore dépigmentante.

### Exemple 6 de l'invention

### Essai in vivo de l'activité anti tyrosinase de la composition de l'invention de l'exemple

Une évaluation de l'activité anti tyrosinase de la composition décrite à l'exemple 5 est effectuée à l'aide d'un modèle tissulaire ex-vivo sur explant de peau humain. Dans ce modèle, l'évaluation de l'activité tyrosinase est mise en évidence par une étude histochimique basée sur le traitement de l'explant par une solution de L-Dopa, substrat de la tyrosinase. L'oxydation de la L-Dopa par la tyrosinase produit dans les mélanocytes un pigment noir précurseur de la mélanine aisément détectable par microscopie optique à lumière blanche. L'activité de la tyrosinase est appréciée par l'intensité de la réaction colorimétrique obtenue.

### Principe de l'étude

Les explants de peau humaine proviennent d'une plastie abdominale réalisée chez une femme de 27 ans.

La composition de l'exemple 5 est testée à 0,1%, 1% et 10% (v/v) dans l'eau ultrapure. Un témoin positif et un témoin négatif sont également réalisés en parallèle. Le témoin positif sélectionné pour cet étude est l'hydroquinone à 0,55% (p/v) reconnue pour posséder une très forte activité dépigmentante et répertoriée parmi les actif OTC. Bien que ne possédant pas d'activité anti tyrosinasique, cette molécule inhibe la formation de pigments mélaniques dans les mélanocytes, ce qui permet de recommander son utilisation dans cette étude.

Les différents produits à l'essai sont incubés en présence de L-DOPA (0,05 M) pendant 15 heures à 37°C.

Après fixation et inclusion dans la paraffine, les coupes sont colorées à l'hemalun/éosine/safran et au crésyl violet, puis observées en lumière blanche, par microscopie optique.

Les pigments mélaniques, formes via l'oxydation de la L-DOPA exogène par la tyrosinase endogène, sont observés. L'activité de la tyrosinase est appréciée par l'intensité de la réaction colorimétrique obtenue.

Des clichés photographiques, représentatifs de l'effet observé, ont été réalisés.

L'observation de l'explant témoin, traité à la L-DOPA, permet de mettre en évidence la présence de mélanocytes actifs, c'est-à-dire possédant une activité tyrosinasique, dans la couche basale de l'épiderme du donneur.

Utilisée comme produit de référence, l'hydroquinone à 0,55% (p/v) inhibe totalement la formation de pigments mélaniques. Ce résultat attendu valide l'étude réalisée.

La composition de l'exemple 5, testée à 0,1%, 1% et 10% (v/v) inhibe elle aussi totalement la réaction histochimique.

En conclusion, dans les conditions expérimentales retenues, et aux trois concentrations étudiées, la composition de l'exemple 5 inhibe totalement l'activité enzymatique de la tyrosinase. Cette inhibition est exceptionnellement forte puisque l'utilisation de 0,1% de la composition de l'exemple 5 permet un niveau d'inhibition comparable à celui obtenu avec 0,55% d'hydroquinone, un actif dépigmentant reconnu mais extrêmement cytotoxique.

Ce résultat de l'invention est particulièrement surprenant.

La cytotoxité de la composition de l'invention fait l'objet de l'essai ci-après de l'exemple 7.

### Exemple 7 de l'invention

### Essai de cytotoxicité de la composition dans l'exemple 5 vis-à-vis des mélanocytes

La préparation réalisée dans l'exemple 5 est testée pour sa toxicité vis-à-vis des mélanocytes.

Les mélanocytes de la lignée G361 (mélanome humain) ont été cultivés dans du milieu Mac COY, de chez GIBCO®, complété par 10% (v/v) de sérum de veau foctal, 50 UI/ml de pénicilline, 50 µg/ml de streptomycine et 2 mM de glutamine.

Les cellules ont été ensemencées en plaques de culture de 24 puits à raison de 3,10⁵ cellules par puits. Le produit de l'exemple 5 à différentes concentrations est ajouté directement au milieu de culture. Après 72 heures d'incubation en présence du produit de l'exemple 5, la cytotoxicité est évaluée par dosage des protéines totales intracellulaires.

Les résultats montrent dans tous les cas, une relation entre concentrations utilisées lors de ces tests et cytotoxicité. Ils montrent également que la cytotoxicité du produit de l'exemple 5 vis-à-vis des mélanocytes est comprise entre 1 et 10 mg/ml.

Ces valeurs sont à rapprocher de celles décrites dans la littérature par MAEDA & FUKUDA dans "In vitro effectivenness of several whitening cosmetic components in human melancytes", publié dans J. Soc. Cosmeti. Chem. 42, pages 361-368 (1991) où le calcul de la concentration inhibitrice pour laquelle la moitié des cellules mises en culture ne sont plus vivantes (CI₅₀) montre la très forte cytotoxicité vis-à-vis des mélanocytes humains :
- de l'hydroquinone, qui possède une CI₅₀ de 5,5 10⁻³ mg/ml;
- de l'acide linoléique qui possède une CI₅₀ de 2,8 10⁻³ mg/ml, et
- de l'acide ascorbique qui possède une CI₅₀ de 0,88 mg/ml.

De ce fait, l'invention permet d'obtenir une cytotoxité environ 1000 fois plus faible que celle de l'hydroquinone ou de l'acide linoléique.

La cytotoxicité est inférieure à de celle de l'acide ascorbique.

### Exemple 8 de l'invention

### Etude toxicologiques

L'essai de toxicité orale, d'irritation oculaire et d'irritation cutanée ont été réalisé selon la portée ci-après.

### A. Toxicité orale

Cet essai réalisé selon le protocole établi par la ligne directrice de l'OCDE concernant l'étude de la toxicité orale aiguë publié sous le n° 401 du 24 février 1987.

Des tests ont été effectués sur la composition de l'exemple 5 à des doses maximales de 5 g par kilogramme de poids corporel et n'ont provoqué aucune mortalité.

Le produit de l'invention tel que décrit à l'exemple 5 utilisé pur oralement à des doses inférieures à 5 g/kg ne présente donc pas de toxicité orale anormale.

### B. Irritation oculaire

Le test d'irritation oculaire est réalisé selon un protocole en accord avec la ligne directrice de l'OCDE concernant l'étude de l'effet irritant/corrosif aigu sur les yeux tel que publié dans le n° 405 du 24 février 1987.

Le produit de l'invention tel que décrit dans l'exemple 5 instillé pur est apparu non irritant pour les yeux au sens de la Directive 91/326 CEE.

### C. Irritation cutanée

Cet essai d'irritation cutanée a été réalisé selon le protocole en accord avec la ligne directrice de l'OCDE concernant l'étude de l'effet irritant/corrosif aigu sur la peau publié au n° 404 du 17 juillet 1992.

Le produit de l'invention tel que décrit dans l'exemple 5, appliqué pur est apparu non irritant pour la peau au sens de la Directive 91/326 CEE.

### D. Tests d'hypoallergénicité

Des tests de maximalisation ont été réalisés selon un protocole adapté de la méthode décrite par Magnusson & Kligman (J. Invest. Derm. 52, 268-276, 1969).

La composition de l'exemple 5, utilisée pure, n'a provoqué aucune réaction significative de sensibilisation. Cette composition est ainsi considérée comme étant hypoallergénique, classe I.

### Exemple 9 de l'invention

### Utilisation des produits de l'invention dans des formulations cosmétiques émulsion eau-dans-huile à usage cosmétique ou pharmaceutique

On prépare une émulsion eau-dans-huile à partir des quatre groupes de composants suivants A, B, C, D en pourcentage en poids :

| Groupe A | |
|---|---|
| Butylèneglycol | 2 |
| Glycine | 3 |
| Sodium dihydroxycétylphosphaste, isopropyldihydroxycétyléther | 2 |
| Eau | qsp 100 |

| Groupe B | |
|---|---|
| Glycostéarate Se | 14 |
| Triisononanoine | 5 |
| Cocoate d'octyle | 6 |

| Groupe C | |
|---|---|
| Butylèneglycol | 2 |
| Mélange de parabènes sous forme de méthyle, éthyle et propyle parabènes | 2 |
| pH ajusté à 5,5 avec un acide fort ou faible dilué | |

| Groupe D | |
|---|---|
| Composition de l'invention de l'exemple 5 | 0.01-10 % |

On commence à mélanger les composants du groupe A en chauffant tout en agitant jusqu'à une température d'environ 75 %. Les éléments de la phase B sont également séparément mélangés et chauffés à la même température d'environ 75°C. La phase B est introduite dans la phase A sous agitation vigoureuse et les composants des groupes C et D sont ensuite successivement rajoutés après avoir refroidi à environ 30°C. Pour le groupe C, le pH est ajusté à 5,5 avec un acide dilué dans l'eau, tel qu'acide citrique.

Cette composition peut être appliquée directement sur des taches cutanées par l'intermédiaire d'un pinceau ou d'un applicateur pour les faire disparaître progressivement, effet de disparition qui peut être généralement observé après environ une quinzaine de jours. On peut aussi appliquer, plus généralement, sur des zones cutanées sur lesquelles on veut inhiber la mélanogénèse ou dépigmenter.

### Exemple 10 de l'invention

### Utilisation des produits de l'invention dans des formulations cosmétiques sous forme d'émulsion huile-dans-eau à usage cosmétique ou pharmaceutique

Comme pour l'exemple précédent, une émulsion de type huile-dans-eau est préparée à partir des 5 groupes de composants suivants A, B, C, D et E:

| Groupe A | |
|---|---|
| PEG 30-dipolyhydroxystéarate | 3 |
| Triglycéride capriques | 3 |
| Octanoate de cétéaryle | 4 |
| Adipate de dibutyle | 3 |
| Huile de grains de raisins | 1,5 |
| Huile de jojoba | 1,5 |
| Phénoxyéthanol en mélange avec des parabènes sous forme de méthyle, d'éthyle, de propyle et de butyle parabenes | 0,5 |

| Groupe B | |
|---|---|
| Glycérine | 3 |
| Butylèneglycol | 3 |
| Sulfate de magnésium | 0.5 |
| EDTA | 0.05 |
| Eau | qsp 100 |

| Groupe C | |
|---|---|
| Cyclométhicone | 1 |
| Diméthicone | 1 |

| Groupe D | |
|---|---|
| Pafum | 0,3 |

| Groupe E | |
|---|---|
| Composition de l'invention de l'exemple 5 | 0,01-10 % |

Les composants du groupe A sont mélangés ensemble en les ajoutant successivement dans l'ordre indiqué tout en les chauffant jusqu'à environ 75 %. On procède séparément pour les composants du groupe B en les chauffant aussi à une température d'environ 75°C. Ensuite, le groupe B est introduit dans le groupe A sous agitation vigoureuse, puis on laisse refroidir jusqu'à la température ambiante et enfin on ajoute les composants des groupes C, D et E lorsque la température est d'environ 30°C.

Cette composition peut être appliquée directement sur des taches cutanées par l'intermédiaire d'un pinceau ou d'un applicateur pour les faire disparaître progressivement, effet de disparition qui peut être généralement observé après environ une quinzaine de jours. On peut aussi appliquer, plus généralement, sur des zones cutanées sur lesquelles on veut inhiber la mélanogénèse ou dépigmenter.

### Exemple 11 de l'invention

### Composition cosmétique ou pharmaceutique sous forme de shampooing ou de gel douche

On prépare une composition cosmétique sous forme de shampooing ou de gel douche comprenant une combinaison des 5 groupes de composants suivants A, B, C, D et E en pourcentage en poids :

| Groupe A | |
|---|---|
| Gomme de xanthane | 0,8 |
| Eau | qsp 100 |

| Groupe B | |
|---|---|
| Phénoxyéthanol en mélange avec des parabènes comprenant du méthyle, de l'éthyle, du propyle et du butyle parabènes | 0,5 |
| Butylèneglycol en mélange avec des parabènes comprenant un mélange de méthyle, éthyle et propyle parabènes | 0,5 |

| Groupe C | |
|---|---|
| Acide citrique | 0,8 |

| Groupe D | |
|---|---|
| Sodium laureth sulfate | 40,0 |

| Groupe E | |
|---|---|
| Composition de l'exemple 5 de l'invention | 0,01-10 % |

On procède tout d'abord à la dissolution de la gomme de xanthane dans l'eau à la température ambiante, puis un chauffage progressifjusqu'à atteindre une température d'environ 75°C et on laisse refroidir jusqu'à la température ambiante.

On rajoute ensuite les composants préalablement mélangés du groupe B, puis du groupe C, puis le groupe D et enfin le groupe E.

On peut utiliser cette composition qui se présente sous forme de shampooing ou de gel douche sur toutes les zones du corps que l'on souhaite traiter pour un effet d'inhibition de mélanogénèse ou de dépigmentation.

### Exemple 12 de l'invention

### Composition cosmétique ou pharmaceutique selon l'invention sous forme de gel agueux de type contour de l'oeil, un gel pour le corps

Cette composition est préparée en mélangeant les ingrédients actifs suivants, en pourcentage en poids :

| | |
|---|---|
| Composition de l'invention de l'exemple 5 | 0,01-10 |
| Carbomer | 0,5 |
| Butylèneglycol | 15 |
| Phénoxyéthanol en mélange avec des parabènes incluant des mélanges de méthyle, éthyle, propyle et butyle parabènes | 0,5 |
| Eau | qsp 100 |

Cette composition est préparée de manière classique en mélangeant tout d'abord la composition de l'invention de l'exemple 5 avec le butylèneglycol, le phénoxyéthanol en mélange avec les parabènes, et l'eau puis en ajoutant enfin le carbomer en obtenant ainsi un gel aqueux qui peut être appliqué sur les zones du corps pour lesquelles un effet d'inhibition de la tyrosinase, donc d'inhibition de la mélanogénèse ou dépigmentant est recherché, comme les contours de l'oeil.

Dans cette composition, on disperse le carbomer dans l'eau à température ambiante, puis on chauffe progressivement jusqu'à environ 75 % sous agitation vigoureuse et on maintient à cette température sous agitation pendant environ 30 min jusqu'à formation du gel.

On laisse ensuite refroidir jusqu'à environ 30°C, puis on ajoute le butylèneglycol, puis le phénoxyéthanol dans les parabènes et enfin la composition selon l'invention. Il est à noter que pour chacune des compositions précédentes des exemples 9 à 12, on peut remplacer la composition de l'exemple 5 par tout autre produit selon l'invention.

### Exemple 13 de l'invention

### Test de mise en évidence de l'activité anti-tyrosinase des produits de l'invention incorporés dans des formulations cosmétiques ou pharmaceutiques

Le sulfite de sodium a une concentration de 5 %, comme indiqué à l'exemple 1, tableau II, soit seul, soit en mélange avec 10 % d'extrait de mûrier ou avec 20% d'extrait de citron, ou encore le méthabisulfite de sodium à la concentration de 5 % dans l'eau déminéralisée ont été séparément incorporés à environ 30 % à la formulation en émulsion eau-dans-huile de l'exemple 9, de façon à obtenir une concentration de 1 % dans cette formulation.

Après refroidissement à la température ambiante, l'activité anti-tyrosinase des formulations est évaluée.

Pour ce faire, 100 g d'une solution aqueuse de 20 % de NaCl sont ajoutés à 100 g de chacune des formulations cosmétiques ou pharmaceutique qui ont été obtenues avec les produits de l'invention. L'ensemble obtenu a été homogénéisé pendant 10 min sous agitation très forte, puis centrifugé à 6 000 rpm pendant 60 min.

La phase aqueuse est récupérée et l'activité anti-tyrosinase est détectée par la méthode décrite dans l'exemple 1.

L'activité anti-tyrosinase telle qu'évaluée sur les produits de l'invention de l'exemple 1 incorporés dans des formulations cosmétiques ou pharmaceutiques a été détectée pour chacun d'entre eux à 95-100 %.

La même évaluation d'activité anti-tyrosinase est réalisée 30 jours après, 60 jours après et 90 jours après la fabrication.

Aucune diminution de l'activité anti-tyrosinase n'a pu être observée, ce qui montre la très grande stabilité de l'inhibition de la tyrosinase.

Naturellement, l'invention comprend tous les équivalents techniques des moyens décrits ainsi que leur diverses combinaisons.

## Revendications

1. Utilisation de sulfites, incluant les métabisulfites, comme agent cosmétique à activité inhibitrice de la mélanogénèse, ou à activité dépigmentante; en combinaison avec au moins un extrait végétal en une quantité suffisante pour diminuer ou empêcher l'apparition d'odeur.

2. Utilisation de sulfites, incluant les métabisulfites, pour la fabrication de compositions pharmaceutiques, et notamment dermatologiques, à activité d'inhibition de la mélanogénèse, ou à activité dépigmentante; en combinaison avec au moins un extrait végétal en une quantité suffisante pour diminuer ou empêcher l'apparition d'odeur.

3. Utilisation selon la revendication 1 ou 2, **caractérisée en ce que** le sulfite précité inclut tout sulfite et en particulier des sels de sulfites, en particulier avec des métaux alcalins ou alcalinoterreux, de préférence avec des métaux alcalins tel que le sodium ou le potassium, ainsi que les sels partiels de sulfites comme métabisulfite ou hydrogénosulfite avec des métaux alcalins ou alcalinoterreux, de préférence avec des métaux alcalins et en particulier le sodium ou le potassium.

4. Utilisation selon l'une des revendications précédentes, **caractérisée en ce que** la proportion d'incorporation du sulfite est comprise entre 0,001% et 20%.

5. Utilisation selon l'une des revendications 1 à 4, **caractérisée en ce que** la proportion d'incorporation du sulfite est comprise entre 0,001% et 10% en poids.

6. Utilisation selon l'une des revendications 1 à 5, **caractérisée en ce que** l'extrait végétal précité est choisi parmi le groupe consistant d'un extrait de mûrier, d'un extrait de citron, d'un extrait de saxifrage, d'un extrait de pamplemousse, d'un extrait de raisin, et d'un extrait d'oughon ou d'un mélange quelconque de ces extraits, de façon à diminuer ou à empêcher l'apparition d'odeur.

7. Utilisation selon l'une des revendications 1 à 6, **caractérisée en ce que** la proportion en extrait végétal est comprise entre 0,01% et 90% du poids total de la composition.

8. Utilisation selon l'une des revendications 1 à 7, **caractérisée en ce que** la proportion en extrait végétal est comprise entre 5% et 70% du poids total de la composition.

9. Utilisation selon l'une des revendications 1 à 8, **caractérisée en ce que** chaque extrait végétal est présent en des proportions d'au moins 5% et pas plus de 30%, du poids total de la composition.

10. Utilisation selon l'une des revendications précédentes, **caractérisée en ce qu'**il est préparé une composition cosmétique ou pharmaceutique, notamment dermatologique, ayant un pH compris entre 5 et 8.

11. Solution de 5% en poids de sulfite de sodium avec 10% en poids d'un extrait de mûrier, la solution présentant un pH de 5,5 ou 6,5.

12. Solution de 5% en poids de sulfite de sodium avec 20% en poids d'un extrait de citron, la solution présentant un pH de 5,5 ou 6,5.

13. Solution de 5% en poids de métabisulfite de sodium avec 20% en poids d'un extrait de saxifrage, la solution présentant un pH de 5,5 ou 6,5.

14. Solution de 5% en poids de métabisulfite de sodium avec 10% en poids d'un extrait de pamplemousse, la solution présentant un pH de 5,5 ou 6,5.

15. Solution de 5% en poids de sulfite de potassium avec 20% en poids d'un extrait de raisin, la solution présentant un pH de 5,5 ou 6,5.

16. Solution de 5% en poids de sulfite de potassium avec 10% en poids d'un extrait d'oughon, la solution présentant un pH de 5,5 ou 6,5.

17. Solution de 5% en poids de métabisulfite de potassium avec 10% en poids d'un extrait de saxifrage, la solution présentant un pH de 5,5 ou 6,5.

18. Solution de 5% en poids de métabisulfite de potassium avec 5% en poids d'un extrait d'oughon et 10% en poids d'un extrait de pamplemousse, la solution présentant un pH de 5,5 ou 6,5.

19. Composition cosmétique ou pharmaceutique constituée de 5% en poids de sulfite de sodium, 5% en poids de métabisulfite de sodium, 25% en poids d'un extrait de saxifrage, 30% en poids d'un extrait de raisin, 5% en poids d'un extrait de mûrier, 5% en poids d'un extrait d'oughon, 0,5% en poids d'EDTA et de qsp 100% en poids d'eau déminéralisée , la composition présentant un pH de 5,5 ou 6,5.

20. Composition de base utile pour la préparation d'une composition cosmétique ou pharmaceutique, la composition de base étant constituée de 5% en poids de sulfite de sodium, 5% en poids de métabisulfite de sodium, 25% en poids d'un extrait de saxifrage, 30% en poids d'un extrait de raisin, 5% en poids d'un extrait de mûrier, 5% en poids d'un extrait d'oughon, 0,5% en poids d'EDTA et de qsp 100% en poids d'eau déminéralisée, la composition de base présentant un pH de 5,5 ou 6,5.

21. Procédé cosmétique d'inhibition de la mélanogénèse ou de dépigmentation, **caractérisé en ce qu'**il comprend l'application sur les zones concernées d'un animal, avantageusement d'un être humain, de manière topique, d'une composition contenant comme principe actif inhibiteur de la mélanogénèse ou dépigmentation, une quantité efficace dans ce but d'au moins un sulfite, incluant un métabisulfite, en combinaison avec au moins un extrait végétal en quantité suffisante pour diminuer ou empêcher l'apparition d'odeur.

22. Procédé selon la revendication 21, **caractérisé en ce que** le sulfite inclut tout sulfite et en particulier les sels partiels de sulfite comme un métabisulfite ou hydrogénosulfite, avec des métaux alcalins ou alcalinoterreux, de préférence avec des métaux alcalins et en particulier le sodium ou le potassium.

23. Procédé selon la revendication 21 ou 22, **caractérisé en ce que** le sulfite précité est utilisé dans une composition à une proportion d'incorporation comprise entre 0,001% et 20%, avantageusement entre 0,01% et 10% en poids.

24. Procédé selon l'une des revendications 21 à 23, **caractérisé en ce que** le pH de ladite composition est compris entre 5 et 8.

25. Procédé selon l'une des revendications 21 à 24, **caractérisé en ce que** l'extrait végétal est choisi parmi le groupe consistant d'un extrait de mûrier, d'un extrait de citron, d'un extrait de saxifrage, d'un extrait de pamplemousse, d'un extrait de raisin, et d'un extrait d'oughon ou d'un mélange quelconque de ces extraits, de façon à diminuer ou à empêcher l'apparition d'odeur.

26. Procédé selon l'une des revendications 21 à 25, **caractérisé en ce que** la proportion en extrait végétal est comprise entre 0,01% et 90%, mieux entre 5% et 70% du poids total de la composition.

27. Procédé selon l'une des revendications 21 à 26, **caractérisé en ce qu'**on utilise la composition de la revendication 19 ou 20.

## Claims

1. Use of sulfites, including metabisulfites, as cosmetic agents with melanogenesis-inhibiting activity or with depigmenting activity, in combination with at least one plant extract in an amount sufficient to reduce or prevent the appearance of odour.

2. Use of sulfites, including metabisulfites, for the manufacture of pharmaceutical compositions, especially dermatological compositions, with melanogenesis-inhibiting activity or with depigmenting activity, in combination with at least one plant extract in an amount sufficient to reduce or prevent the appearance of odour.

3. Use according to claim 1 or 2, **characterized in that** said sulfite includes any sulfite and particularly sulfite salts, especially those with alkali metals or alkaline earth metals and preferably those with alkali metals such as sodium or potassium, as well as partial sulfite salts, such as metabisulfites or dithionites, with alkali metals or alkaline earth metals, preferably those with alkali metals and particularly those with sodium or potassium.

4. Use according to one of the preceding claims, **characterized in that** the sulfite is incorporated in a proportion of between 0.001% and 20%.

5. Use according to one of claims 1 to 4, **characterized in that** the sulfite is incorporated in a proportion of between 0.001% and 10% by weight.

6. Use according to one of claims 1 to 5, **characterized in that** said plant extract is selected from the group consisting of a mulberry extract, a lemon extract, a saxifrage extract, a grapefruit extract, a grape extract and an oughon extract, or any mixture of these extracts, for the purpose of reducing or preventing the appearance of odour.

7. Use according to one of claims 1 to 6, **characterized in that** the proportion of plant extract is between 0.01% and 90% of the total weight of the composition.

8. Use according to one of claims 1 to 7, **characterized in that** the proportion of plant extract is between 5% and 70% of the total weight of the composition.

9. Use according to one of claims 1 to 8, **characterized in that** each plant extract is present in proportions of at least 5% and no more than 30% of the total weight of the composition.

10. Use according to one of the preceding claims, **characterized in that** a cosmetic or pharmaceutical composition, especially a dermatological composition, with a pH of between 5 and 8 is prepared.

11. Solution of 5% by weight of sodium sulfite with 10% by weight of a mulberry extract, the solution having a pH of 5.5 or 6.5.

12. Solution of 5% by weight of sodium sulfite with 20% by weight of a lemon extract, the solution having a pH of 5.5 or 6.5.

13. Solution of 5% by weight of sodium metabisulfite with 20% by weight of a saxifrage extract, the solution having a pH of 5.5 or 6.5.

14. Solution of 5% by weight of sodium metabisulfite with 10% by weight of a grapefruit extract, the solution having a pH of 5.5 or 6.5.

15. Solution of 5% by weight of potassium sulfite with 20% by weight of a grape extract, the solution having a pH of 5.5 or 6.5.

16. Solution of 5% by weight of potassium sulfite with 10% by weight of an oughon extract, the solution having a pH of 5.5 or 6.5.

17. Solution of 5% by weight of potassium metabisulfite with 10% by weight of a saxifrage extract, the solution having a pH of 5.5 or 6.5.

18. Solution of 5% by weight of potassium metabisulfite with 5% by weight of an oughon extract and 10% by weight of a grapefruit extract, the solution having a pH of 5.5 or 6.5.

19. Cosmetic or pharmaceutical composition consisting of 5% by weight of sodium sulfite, 5% by weight of sodium metabisulfite, 25% by weight of a saxifrage extract, 30% by weight of a grape extract, 5% by weight of a mulberry extract, 5% by weight of an oughon extract, 0.5% by weight of EDTA, and demineralized water ad 100% by weight, the composition having a pH of 5.5 or 6.5.

20. Base composition useful for the preparation of a cosmetic or pharmaceutical composition, the base composition consisting of 5% by weight of sodium sulfite, 5% by weight of sodium metabisulfite, 25% by weight of a saxifrage extract, 30% by weight of a grape extract, 5% by weight of a mulberry extract, 5% by weight of an oughon extract, 0.5% by weight of EDTA, and demineralized water ad 100% by weight, the composition having a pH of 5.5 or 6.5.

21. Cosmetic method of inhibiting melanogenesis or depigmentation, **characterized in that** it comprises the topical application, to the appropriate areas of an animal, advantageously a human being, of a composition containing, as the active principle for inhibiting melanogenesis or depigmentation, at least one sulfite, including a metabisulfite, in an amount effective for this purpose, in combination with at least one plant extract in an amount sufficient to reduce or prevent the appearance of odour.

22. Method according to claim 21, **characterized in that** the sulfite includes any sulfite and particularly partial sulfite salts, such as metabisulfites or dithionites, with alkali metals or alkaline earth metals, preferably those with alkali metals and particularly those with sodium or potassium.

23. Method according to claim 21 or 22, **characterized in that** said sulfite is incorporated into a composition in a proportion of between 0.001% and 20% and advantageously of between 0.01% and 10% by weight.

24. Method according to one of claims 21 to 23, **characterized in that** the pH of said composition is between 5 and 8.

25. Method according to one of claims 21 to 24, **characterized in that** the plant extract is selected from the group consisting of a mulberry extract, a lemon extract, a saxifrage extract, a grapefruit extract, a grape extract and an oughon extract, or any mixture of these extracts, for the purpose of reducing or preventing the appearance of odour.

26. Method according to one of claims 21 to 25, **characterized in that** the proportion of plant extract is between 0.01% and 90% and preferably between 5% and 70% of the total weight of the composition.

27. Method according to one of claims 21 to 26, **characterized in that** the composition of claim 19 or 20 is used.

## Patentansprüche

1. Verwendung von Sulfiten, einschließlich Metabisulfiten, als kosmetisches Mittel mit Melanogenese-hemmender Aktivität oder mit depigmentierender Aktivität in Kombination mit mindestens einem pflanzlichen Extrakt in einer zur Verringerung oder Verhinderung des Auftretens von Geruch ausreichenden Menge.

2. Verwendung von Sulfiten, einschließlich Metabisulfiten, zur Herstellung von pharmazeutischen, insbesondere dermatologischen Zusammensetzungen mit Melanogenese-hemmender Aktivität oder mit depigmentierender Aktivität in Kombination mit mindestens einem pflanzlichen Extrakt in einer zur Verringerung oder Verhinderung des Auftretens von Geruch ausreichenden Menge.

3. Verwendung nach Anspruch 1 oder 2, **dadurch gekennzeichnet, dass** das Sulfit Ganzsulfit und insbesondere Sulfitsalze, insbesondere mit Alkali- oder Erdalkalimetallen, vorzugsweise mit Alkalimetallen wie Natrium oder Kalium, sowie Sulfit-Teilsalze, wie Metabisulfit oder Hydrogensulfit, mit Alkali- oder Erdalkalimetallen, vorzugsweise Alkalimetallen und insbesondere Natrium oder Kalium enthält.

4. Verwendung nach einem der vorhergehenden Ansprüche, **dadurch gekennzeichnet, dass** das Einarbeitungsverhältnis von Sulfit zwischen 0,001 % und 20 % beträgt.

5. Verwendung nach einem der Ansprüche 1 bis 4, **dadurch gekennzeichnet, dass** das Einarbeitungsverhältnis von Sulfit zwischen 0,001 Gew.% und 10 Gew.% beträgt.

6. Verwendung nach einem der Ansprüche 1 bis 5, **dadurch gekennzeichnet, dass** der pflanzliche Extrakt ausgewählt ist aus der Gruppe bestehend aus einem Maulbeerbaumextrakt, einem Zitronenextrakt, einem Steinbrechextrakt, einem Pampelmusenextrakt, einem Traubenextrakt, einem Oughon-Extrakt oder einer Mischung dieser Extrakte, so dass das Auftreten von Geruch verringert oder verhindert wird.

7. Verwendung nach einem der Ansprüche 1 bis 6, **dadurch gekennzeichnet, dass** der Anteil an pflanzlichem Extrakt zwischen 0,01 % und 90 % des Gesamtgewichts der Zusammensetzung ausmacht.

8. Verwendung nach einem der Ansprüche 1 bis 7, **dadurch gekennzeichnet, dass** der Anteil an pflanzlichem Extrakt zwischen 5 % und 70 % des Gesamtgewichts der Zusammensetzung ausmacht.

9. Verwendung nach einem der Ansprüche 1 bis 8, **dadurch gekennzeichnet, dass** jeder pflanzliche Extrakt in Mengenverhältnissen von mindestens 5 % und nicht mehr als 30 % des Gesamtgewichts der Zusammensetzung vorliegt.

10. Verwendung nach einem der vorhergehenden Ansprüche, **dadurch gekennzeichnet, dass** eine kosmetische oder pharmazeutische, insbesondere dermatologische Zusammensetzung mit einem pH-Wert zwischen 5 und 8 hergestellt wird.

11. Lösung aus 5 Gew.% Natriumsulfit mit 10 Gew.% eines Maulbeerbaumextrakts, wobei die Lösung einen pH-Wert von 5,5 oder 6,5 aufweist.

12. Lösung aus 5 Gew.% Natriumsulfit mit 20 Gew.% eines Zitronenextrakts, wobei die Lösung einen pH-Wert von 5,5 oder 6,5 aufweist.

13. Lösung aus 5 Gew.% Natriummetabisulfit mit 20 Gew.% eines Steinbrechextrakts, wobei die Lösung einen pH-Wert von 5,5 oder 6,5 aufweist.

14. Lösung aus 5 Gew.% Natriummetabisulfit mit 10 Gew.% eines Pampelmusenextrakts, wobei die Lösung einen pH-Wert von 5,5 oder 6,5 aufweist.

15. Lösung aus 5 Gew.% Kaliumsulfit mit 20 Gew.% eines Traubenextrakts, wobei die Lösung einen pH-Wert von 5,5 oder 6,5 aufweist.

16. Lösung aus 5 Gew.% Kaliumsulfit mit 10 Gew.% eines Oughon-Extrakts, wobei die Lösung einen pH-Wert von 5,5 oder 6,5 aufweist.

17. Lösung aus 5 Gew.% Kaliummetabisulfit mit 10 Gew.% eines Steinbrechextrakts, wobei die Lösung einen pH-Wert von 5,5 oder 6,5 aufweist.

18. Lösung aus 5 Gew.% Kaliummetabisulfit mit 5 Gew.% eines Oughon-Extrakts und 10 Gew.% eines Pampelmusenextrakts, wobei die Lösung einen pH-Wert von 5,5 oder 6,5 aufweist.

19. Kosmetische oder pharmazeutische Zusammensetzung, bestehend aus 5 Gew.% Natriumsulfit, 5 Gew.% Natriummetabisulfit, 25 Gew.% eines Steinbrechextrakts, 30 Gew.% eines Traubenextrakts, 5 Gew.% eines Maulbeerbaumextrakts, 5 Gew.% eines Oughon-Extrakts, 0,5 Gew.% EDTA und q.s.p. 100 Gew.% entmineralisiertem Wasser, wobei die Zusammensetzung einen pH-Wert von 5,5 oder 6,5 aufweist.

20. Basiszusammensetzung, die zur Herstellung einer kosmetischen oder pharmazeutischen Zusammensetzung nützlich ist, wobei die Basiszusammensetzung aus 5 Gew.% Natriumsulfit, 5 Gew.% Natriummetabisulfit, 25 Gew.% eines Steinbrechextrakts, 30 Gew.% eines Traubenextrakts, 5 Gew.% eines Maulbeerbaumextrakts, 5 Gew.% eines Oughon-Extrakts, 0,5 Gew.% EDTA und q.s.p. 100 Gew.% entmineralisiertem Wasser besteht und einen pH-Wert von 5,5 oder 6,5 aufweist.

21. Kosmetisches Verfahren zur Hemmung von Melanogenese oder zur Depigmentierung, **dadurch gekennzeichnet, dass** es die Anwendung einer Zusammensetzung auf den betreffenden Stellen eines Lebewesens, vorzugsweise eines Menschen, auf topische Weise umfasst, welche Zusammensetzung als Wirkstoff zur Melanogenese-Hemmung oder Depigmentierung eine für diesen Zweck wirksame Menge mindestens eines Sulfits, einschließlich eines Metabisulfits, in Kombination mit mindestens einem pflanzlichen Extrakt in zur Verringerung oder Verhinderung des Auftretens von Geruch ausreichender Menge enthält.

22. Verfahren nach Anspruch 21, **dadurch gekennzeichnet, dass** das Sulfit Ganzsulfit und insbesondere Sulfit-Teilsalze, wie Metabisulfit oder Hydrogensulfit, mit Alkali- oder Erdalkalimetallen, vorzugsweise mit Alkalimetallen und insbesondere Natrium oder Kalium, enthält.

23. Verfahren nach Anspruch 21 oder 22, **dadurch gekennzeichnet, dass** das Sulfit in einer Zusammensetzung in einem Einarbeitungsverhältnis zwischen 0,001 Gew.% und 20 Gew.%, vorteilhaft zwischen 0,01 Gew.% und 10 Gew.%, verwendet wird.

24. Verfahren nach einem der Ansprüche 21 bis 23, **dadurch gekennzeichnet, dass** der pH-Wert der Zusammensetzung zwischen 5 und 8 liegt.

25. Verfahren nach einem der Ansprüche 21 bis 24, **dadurch gekennzeichnet, dass** der pflanzliche Extrakt ausgewählt ist aus der Gruppe bestehend aus einem Maulbeerbaumextrakt, einem Zitronenextrakt, einem Steinbrechextrakt, einem Pampelmusenextrakt, einem Traubenextrakt, einem Oughon-Extrakt oder einer Mischung dieser Extrakte, so dass das Auftreten von Geruch verringert oder verhindert wird.

26. Verfahren nach einem der Ansprüche 21 bis 25, **dadurch gekennzeichnet, dass** der Anteil an pflanzlichem Extrakt zwischen 0,01 % und 90 %, besser zwischen 5 % und 70 % des Gesamtgewichts der Zusammensetzung ausmacht.

27. Verfahren nach einem der Ansprüche 21 bis 26, **dadurch gekennzeichnet, dass** die Zusammensetzung der Ansprüche 19 oder 20 verwendet wird.
